# EUROPÄISCHE PATENTSCHRIFT

(11) **EP 1 089 792 B1**
(45) Veröffentlichungstag und Bekanntmachung des Hinweises auf die Patenterteilung: **17.05.2006**
(21) Anmeldenummer: 99938182.5
(22) Anmeldetag: 12.06.1999
(51) Int. Cl.: A61N 2/02

(54) **VORRICHTUNG ZUR BEHANDLUNG MIT MAGNETISCHEN FELDERN**
DEVICE FOR TREATMENT WITH MAGNETIC FIELDS
DISPOSITIF DE TRAITEMENT PAR DES CHAMPS MAGNETIQUES

(30) Priorität: 22.06.1998 DE 19827736
(43) Veröffentlichungstag der Anmeldung: 11.04.2001
(73) Patentinhaber: Muntermann, Axel, 35578 Wetzlar (DE)
(72) Erfinder: Muntermann, Axel, 35578 Wetzlar (DE)
(74) Vertreter: Herden, Andreas F.
(86) Internationale Anmeldenummer: PCT/DE1999/001722
(87) Internationale Veröffentlichungsnummer: WO 1999/066986

(56) Entgegenhaltungen:
- EP-A- 0 366 158
- WO-A-97/46277
- DE-A- 2 821 114
- DE-A- 4 026 173
- US-A- 4 428 366
- US-A- 5 050 605
- US-A- 5 224 922
- US-A- 5 690 109

## Beschreibung

Die Erfindung betrifft eine Vorrichtung zur Behandlung mit magnetischen Feldern gemäß den Merkmalen des Anspruch 1.

Magnetfelderzeugende Geräte werden seit längerem in der Medizin eingesetzt. Sie lassen sich zunächst in zwei Gruppen unterteilen, nämlich solchen für die Diagnose und solchen für die therapeutische Behandlung.

Aus dem US-Patent 5,050,605 (Eydelman et al.) sind Bildantennen mit zwei sich gegenüberliegenden Spiralspulen bekannt. Diese Bildantennen dienen dem Empfang der bei der Kernspinresonanz- (NMR) Diagnose zu erzeugenden Bildinformation. Ein weiteres NMR-Abbildungsverfahren ist aus der EP 0 366 158 B1 bekannt.

Aus dem US-Patent 5,690,109 (Govind et al.) ist ein Verfahren zur nichtinvasiven Hyperthermie von Gewebe und Organismen mittels Kernspinresonanz bekannt. Ziel dieses Verfahrens ist es jedoch lediglich Krebszellen durch die Hyperthermie zu zerstören. Eine positive therapeutische Beeinflussung von lebendem Gewebe ist nicht erwähnt.

Ferner sind Vorrichtungen bekannt, die unter Anwendung gepulster oder modulierter Magnetfelder positiven Einfluß auf biologisches Gewebe nehmen. Dabei wird, wie beispielhaft aus der DE 40 26 173 zu entnehmen ist, das Gewebe einem konstanten Magnetfeld und einem diesem überlagerten magnetischen Wechselfeld ausgesetzt. Es konnte im Rahmen der Anwendung derartiger Geräte gezeigt werden, daß durch das Bestrahlen von biologischem Gewebe mit Magnetfeldern bzw. mit magnetischen Wechselfeldern eine positive therapeutische Wirkung erzielt werden kann. Die Heilwirkung solcher Magnetfeld-Therapiegeräte besteht unter anderem in der Linderung von Osteoporose oder den Folgen eines Schlaganfalls. Dabei erscheint es als wahrscheinlich, daß durch die angewandten Magnetfelder, Transport- und/oder Stoffwechselprozesse gefördert werden, die zu einer positiven therapeutischen Wirkung führen. Bislang ging man davon aus, daß der oben beschriebene Prozeß, durch die Anregung bzw. die Absorption von Ionen-Zyklotron-Resonanzen (IZR) in einem biologischen Körper verursacht wird. Dies erscheint jedoch unter Umständen fraglich, da Zyklotronresonanzen im Allgemeinen nur an freien Teilchen auftreten, wie beispielsweise im Vakuum oder bei Elektronen im Leitungsband eines Halbleiters. Ferner kann auch durch einfache Rechnung gezeigt werden, daß sich eine Zyklotron-Bewegung auf einer Kreisbahn vollziehen wurde, deren Radius bereits den durchschnittlichen Durchmesser eines Querschnitts eines menschlichen Körpers übersteigt. Dies bedeutet, daß für den Energieübertrag eine Erklärung hinsichtlich einer Zyklotronresonanz insbesondere bei festem Gewebe fraglich sein kann. In der Praxis hat sich gezeigt, daß man bei den bestehenden Magnetfeld-Therapie-Geräten vielfach gezwungen ist zunächst mehrere Behandlungen durchzuführen, um so die Frequenzen für die nachfolgenden Behandlungen festzulegen, die den erwünschten positiven Effekt verursachen. Eine Vorgehensweise, die die Therapie mit Magnetfelder sehr aufwendig und unpräzise gestaltet und eine gezielte Vorgehensweise bei definierbaren und lokalierbaren Beschwerden bei fester oder flüssiger Materie nur ausnahmsweise oder gar nicht zuläßt.

Ein weiteres Verfahren zur Erzielung eines regerativen Effektes in einem biologischen System mittels eines elektromagnetischen Feldes ist aus dem US-Patent 5,224,922 bekannt. Diese geht aber ebenfalls von Zyklotronresonanzbedingungen aus.

Der Erfindung liegt daher die Aufgabe zu Grunde eine Vorrichtung zur Behandlung mit magnetischen Feldern bereitzustellen, die die benannten Nachteile vermeidet und es insbesondere erstmals erlaubt auf eine gezielte reproduzierbare Weise Behandlungen mit Magnetfeldern in allen biologischen Materien vorzunehmen, unabhängig davon, ob ionische Teile vorhanden sind.

Die Lösung dieser Aufgabe erfolgt auf höchst überraschende Weise bereits durch die Merkmale des Anspruchs 1. Vorteilhafte und bevorzugte Weiterbildungen sind Gegenstand der Unteransprüche.

Gegenüber den bekannten Lösungsversuchen basiert die erfindungsgemäße Vorrichtung auf dem Gedanken, daß die positive Behandlungswirkung durch das Erzeugen von Spinresonanzen, vorzugsweise wiederholter Spinresonanzsequenzen, erzielbar ist.

In besonders vorteilhafter Weise umfaßt eine bevorzugte Ausführungsform der Erfindung einen Magneten zum Erzeugen eines im wesentlichen konstanten Magnetfeldes und ein Spulensystem zum Erzeugen eines magnetischen Wechselfeldes senkrecht zu dem Magnetfeld des Magneten, wobei dies, für den Fachmann offensichtlich, einer klassischen Anordnung zur Durchführung einer Spinresonanz entspricht. Ferner verfügt die erfindungsgemäße Vorrichtung in vorteilhafter Weiterbildung auch über eine Steuerelektronik zum Ansteuern des Magneten und des Spulensystems, die derart einstellbar ist, so daß in einem zwischen den oben beschriebenen Magneten eingebrachten biologischen Körper eine Kernspinresonanz erzeugt wird. Dabei ist der Steuereinrichtung in sehr vorteilhafter Weise eine Einrichtung zugeordnet, mittels der, über einen festen Behandlungszeitraum, Parameter oder Intervalle festgelegt werden können, nach denen die erfindungsgemäße Vorrichtung, den durch die Steuerelektronik gesteuerten Kernspinresonanzprozeß bzw. Kernspinabsorptionsurozeß wiederholt.

Im Rahmen der Erfindung hat es sich als höchst vorteilhaft herausgestellt, wenn die Wiederholungsrate, d.h. die zeitliche Abfolge der durchgeführten Kernspinresonanzen in Abhängigkeit von der Spin-Gitter-Relaxationszeit T₁ bestimmt wird. Die Spin-Gitter-Relaxationszeit beschreibt als Zeitkonstante den exponentiellen bzw. logarithmischen Aufbau einer makroskopischen Magnetisierung in Richtung eines Magnetfeldes. Durch das resonante Einstrahlen eines magnetischen Wechselfeldes senkrecht zum benannten Magnetfeld baut sich die Magnetisierung in Richtung des Magnetfeldes ab. Wird nach diesem Abbau der Magnetisierung nicht mehr resonant eingestrahlt, so baut sich die Magnetisierung in Magnetfeldrichtung erneut auf, wobei aufgrund des logarithmischen Anwachsens davon ausgegangen werden kann, daß die Magnetisierung nach einer Zeit von ca. 3T₁ ca. 95% des Ausgangswertes der Magnetisierung wieder erreicht. Das heißt, daß nach einem Zeitintervall von 3T₁ ein neuerlicher effektiver Energietransfer durch Spinresonanz erzielbar ist. Daher ist die Festlegung der Wiederholungsrat der Spinresonanz auf die dreifache Spin-Gitter-Relaxationzeit des zu behandelnden Körpers oder Körperteils idealtypisch für den vorliegenden Erfindungsgegenstand, jedoch nicht zwingend, so daß auch andere Frequenzen vorstellbar sind. Zudem ist durch diese Festlegung eine positive biologische Wirkung sofort erzielbar. Es ist im Gegensatz zum Stand der Technik nicht mehr nötig, durch umfangreiche Vorbehandlungen mehr oder minder gezielt die Frequenz zu bestimmen, die eine biologische Wirkung erzielt. Da die Spinresonanz sowohl für gasförmige als auch für in Lösung befindliche und für fest eingebaute Moleküle erfolgen kann, sind vorteilhafterweise auch die entsprechenden bzw. die zur Behandlung benötigten Spingitterrelaxationszeiten durch Messungen zugänglich.

Demzufolge umfaßt die Vorrichtung zur Behandlung mit magnetischen Feldern eine weiteres Spulensystem. Die Achse dieses Spulensystems steht vorzugsweise sowohl senkrecht auf der Achse des Magneten und der des ersten Spulensystems. Dieses Spulensystem wird als Detektionsspulensystem verwendet. Dabei kann vor Behandlungsbeginn mit den Detektionsspulen in höchst vorteilhafterweise, beispielsweise im Rahmen einer Spin-Echo-Messsung, die Bestimmung der Spin-Gitter-Relaxationszeit T₁ erfolgen, mittels der, wie bereits oben beschrieben, die Wiederholungsrate festlegbar ist.

Ferner ist in positiver Weitergestaltung des Erfindungsgegenstandes diesem eine Auswerteeinrichtung zugeordnet, die es ermöglicht aus der oben dargelegten Spin-Echo-Messung den benötigten Parameter T₁ zu bestimmen. Wird zudem auch die bereits beschriebene Detektionsspule bereitgestellt, so kann die Wiederholungsrate für die Spinresonanz direkt und ganz gezielt für den zu behandelnden Körper oder das zu behandelnde Körperteil vor Ort erfolgen. D.h. Behandlungstermine die nur dem Auffinden einer möglichen "Wirkungsfrequenz" dienen sind nicht mehr nötig. Dabei kann jedoch auch in einer vereinfachten Ausführungsform diese Einrichtung vorteilhafterweise nur zur Aufnahme und zum Bereitstellung von bereits bestimmten bzw. aus der Literatur zu entnehmenden Werten für T₁ oder sonstigen Parametern wie z.B. das Magnetfeld B₀, das Wechselfeld B₁ oder die Larmorfrequenz f₀ oder andere entsprechende Parameter dienen, um auf diese Weise die Bedingungen festzulegen unter denen die Behandlung mittels Spinresonanz durchzuführen ist.

Höchst vorteilhaft ist auch, daß die Steuerelektronik eine Meßeinrichtung umfaßt, die in der Lage ist, beispielsweise über angebrachte Detektionspulen wie sie bereits oben beschrieben wurden, die Energie zu messen, die im Laufe einer Abfolge von Kernspinresonanzen an das zu behandelnde Gewebe bzw. den Körperteil abgegeben wird. Auf sehr einfache Art und Weise kann so eine Beziehung hergestellt werden, durch die die Abhängigkeit des Behandlungserfolgs und die an den Körper abgegebene "Energiedosis" darstellbar ist. Entsprechende Aufzeichnungen können insbesondere dazu verwendet werden, um für bestimmte Anwendungen bestimmte "Energiedosen" festzulegen. Ferner ist es aber auch möglich diese zur Überwachung des Krankheitsverlaufs zu verwenden. Wobei für den Fachmann offensichtlich ist, daß derartige Aufzeichnungen auch auf vielfältige andere Art und Weise eingesetzt werden können.

Im Idealfall reicht es bei der Spinresonanz aus eine diskrete Frequenz, nämlich die sog. Larmorfrequenz, eines Wechselfeldes einzustrahlen, um im klassischen Sinne die makroskopische Mangnetisierung umzuklappen bzw. einen entsprechenden energetischen Übergang zu erzielen. In der Praxis ist eine derart diskrete Frequenz nicht zu erzielen. Zudem ist damit zu rechnen, daß das angewandte Magnetfeld nicht homogen ist und sich außerdem auch innerhalb der Bereiche, die mit Spinresonanz behandelt werden, durch chemische Verschiebungen Inhomogenitäten ergeben, so daß die Spins nicht mit einer einheitlichen Larmorfrequenz in Phase präzedieren. Um dennoch möglichst: viele Spins zum Umklappen zu bringen umfaßt daß vom erfindungsgemäßen Spulensystem erzeugte magnetische Wechselfeld vorteilhafterweise eine Vielzahl von definierten Fourier-Komponenten.

In positiver Weiterbildung der erfindungsgemäßen Vorrichtung besteht der Magnet aus einer Helmholtzspulenanordnung. Helmholtzspulen zeichnen sich insbesondere dadurch aus, daß sie in der Lage sind im wesentlichen konstante homogene Magnetfelder zu erzeugen. Darüber hinaus bieten sie den Vorteil, daß mit ihnen eine Feldverschiebung auf einfache Weise durchführbar ist. Diese Feldverschiebung kann zum einen durch die Veränderung des durch die Spulen fließenden Stroms erfolgen oder zum anderen, indem durch eine weitere Spule dem Helmholtzfeld ein zusätzliches Magnetfeld überlagert wird. Es ist daher mit einem Helmholtzspulenpaar auf einfache Weise möglich eine Kernspinresonanz durch Feldverschiebung durchzuführen.

In der Praxis konnte jedoch gezeigt werden, daß in höchst vorteilhafter Weiterbildung der Erfindung eine Helmholtzspulenanordnung so wie sie oben beschrieben wurde nicht zwingend ist. Für die zu Behandlungzwecken durchgeführte Spinresonanz ist es beispielsweise ausreichend, wenn zum Erzeugen des Magnetfeldes ein Hartferrit Magnet verwendet wird. Dadurch daß auch einfachere Magnete eingesetzt werden können, ist es möglich die erfindungsgemäße Vorrichtung wesentlich günstiger herzustellen und somit die Behandlung mit der erfindungsgemäßen Vorrichtung einem großen Kreis an Patienten zukommen zu lassen. In diesem Zusammmenhang konnte vorteilhafter Weise gezeigt werden, daß bei Magnetfeldstärken von ca. 22 Gauß mit einer Abweichung im Feld von bis zu ca. ± 0,6 Gauß für die Behandlungszwecke der vorliegenden Erfindung ausreichen.

Dies ist unter anderem dadurch möglich, daß dem vergleichsweise stark fluktuierenden Feld, mittels einer Hilfsspule ein zusätzliches variables Hilfsfeld überlagert wird. Wobei diese Hilfsspule durch eine Steuereinrichtung derart angesteuert wird, so daß die Kernspinresonanz, bei einem mit konstanter Frequenz eingestrahlten magnetischen Wechselfeld, im schnellen adiabatischen Durchlaufverfahren erfolgt. Der adiabatische Durchlauf beruht unter anderem auf einer Feldverschiebung des durch den Magneten erzeugten Magnetfeldes. Dabei wird in einer möglichen Ausführungsform das Magnetfeld des Magneten durch das Magnetfeld einer Hilfsspule zunächst konstruktiv überlagert, d.h. verstärkt, und am Ende einer Resonanzsequenz kompensierend überlagert, d.h. abgeschwächt. Dies bedeutet, daß während einer Resonanzsequenz ein stetiger Abfall des Magnetfeldes des Magneten erfolgt. Der Abfall des Magnetfeldes besitzt vorzugsweise die Form eines Sägezahns. Beim adiabatischen Durchlauf ist allerdings darauf zu achten, daß die Variation des Feldes nicht zu schnell durchgeführt wird. Betrachtet man nämlich die Magnetfelder bzw. die Magnetisierung in dem mit Larmorfrequenz rotierenden Bezugssystem, so erkennt man, daß aufgrund der Feldüberlagerung innerhalb dieses rotierenden Bezugssystems eine weitere Larmorbewegung einer zusätzlichen Magnetisierung entsteht, die der ursprünglichen durch das Magnetfeld erzeugten Bewegung überlagert ist. Durch die Feldänderung ändert diese zusätzliche Magnetisierung im rotierenden Bezugssystem sein Richtung, da sie sich an dem aus der Überlagerung resultierenden Magnetfeld ausrichtet. Dies findet jedoch nur dann ohne Energieübertrag, d.h. adiabatisch, statt, wenn sich die Änderung das Magnetfeldes hinreichend langsam vollzieht. Hierdurch ist es auf sehr vorteilhafte und kostengünstige Weise möglich sämtliche in der Aktiv-Zone sich befindlichen Körperteile durch die Spinresonanz zu erreichen und einen im wesentlichen optimierten Energieaustausch zu erzielen, da durch die Feldverschiebung des Magnetfeldes, trotz möglicher Abweichung in der Felddichte, die Spins einem geeigneten Magnetfeld ausgesetzt werden, so daß eine Resonanz in jedem Fall mit der quer eingestrahlten Larmorfrequenz entstehen kann.

Im Rahmen der Erfindung liegt ferner die Spinresonanz nicht nur, wie oben beschrieben, durch eine Feldverschiebung durchzuführen, sondern dies auch durch eine reine Frequenzverschiebung zu tun oder den Energietransfer der Spinresonanz mittels einer kombinierten Feld-Frequenz - Verschiebung erfolgen zu lassen. Wobei hierdurch ein schneller adiabatischer Durchlauf nicht ausgeschlossen wird.

Die Erfindung wird nachfolgend anhand bevorzugter Ausführungsformen und unter Bezugnahme auf die beigefügten Zeichnung detailliert beschrieben.

### Es zeigen:

Fig. 1 eine perspektivische Darstellung einer möglichen erfindungsgemäßen Anordnung von Magnetfeldern und Spulen.

Fig. 2 eine perspektivische Darstellung einer weiteren erfindungsgemäßen Anordnung von Magnetfeldern und Spulen.

Fig. 3 ein Blockschaltbild das schematisch die Funktionsgruppen bei einem Einsatz einer erfindungsgemäßen Vorrichtung zur Behandlung mit magnetischen Feldern darstellt.

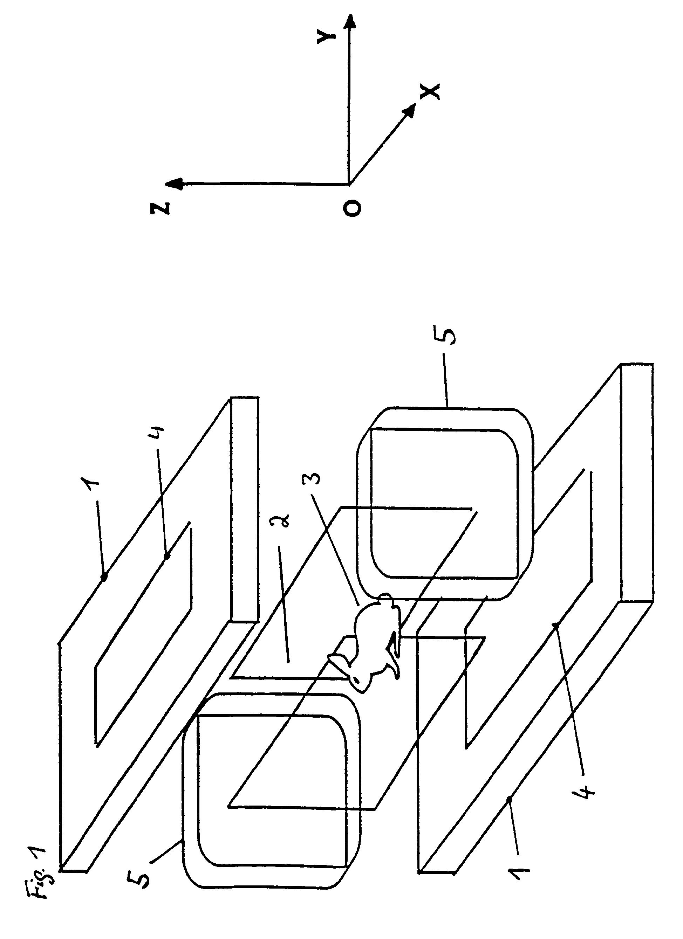

In Fig. 1 ist eine schematische Darstellung einer bevorzugten Anordnung von Spulen bzw. Magneten einer erfindungsgemäßen Vorrichtung. Das rechts von der Anordnung gezeichnete Koordinatensystem soll die räumliche Lage der einzelnen Spulen verdeutlichen. Dabei sind senkrecht zur z-Achse, d.h. senkrecht zur Vertikalen, zwei Pole eines Permanentmagneten 1 angeordnet. Die Feldlinien des durch den Permanentmagneten erzeugten Magnetfeldes verlaufen parallel zur z-Achse. Die beiden Pole 1 des Magneten sind zueinander beabstandet und bilden so eine Zwischenraum 2 aus. Der Zwischenraum ist der sog. Aktiv-Bereich 2 in dem die Magnetfelder bzw. die durchgeführte Spinresonanz ihre Wirkung auf den biologischen Körper 3 entfaltet. Durch den Permanentmagneten 1 wird vorzugsweise ein Magnetfeld von B₀ = 22 Gauß erzeugt, wobei mit Bezug auf die Felddichte im Rahmen des schnellen adiabatischen Durchlaufs eine Felddichtenabweichung von ca. ±0,5 Gauß toleriert werden kann. Ferner ist aus Fig. 1 zu entnehmen, daß dem Permanentmagneten 1 eine Hilfsspule 4 zugeordnet ist. Die Hilfspule 4 dient bei der Durchführung der Spinresonanz dazu, dem im wesentlichen konstanten Magnetfeld ein sich veränderndes Magnetfeld zu überlagern, um im Rahmen des adiabatischen Durchlaufs eine Feldverschiebung durchzuführen. Senkrecht zur z-Achse bzw. den Feldlinien des Permanentmagneten, d.h. entlang der x-Achse, ist eine Sendespulensystem 5 angeordnet. Die Sendespulen 5 erzeugen ein magnetisches Wechselfeld. Beim adiabatischen Durchlauf wird dieses Wechselfeld auf eine im wesentlichen feste Larmorfrequenz eines zu behandelnden biologischen Körpers eingestellt. Wie in Fig. 1 dargestellt, kommt ein weiteres Spulen-System 6 zum Einsatz . Dieses Spulen-System 6 kann zum einen im Rahmen von beispielsweise Spin-Echo-Messungen zur Bestimmung der Spin-Gitter-Relaxationszeit T₁ des zu behandelnden biologischen Körpers eingesetzt werden und zum anderen auch zur Bestimmung der Energie verwendet werden, die durch eine Abfolge von Spinresonanzen an das biologische Objekt in der Aktiv-Zone 2 abgegeben wird.

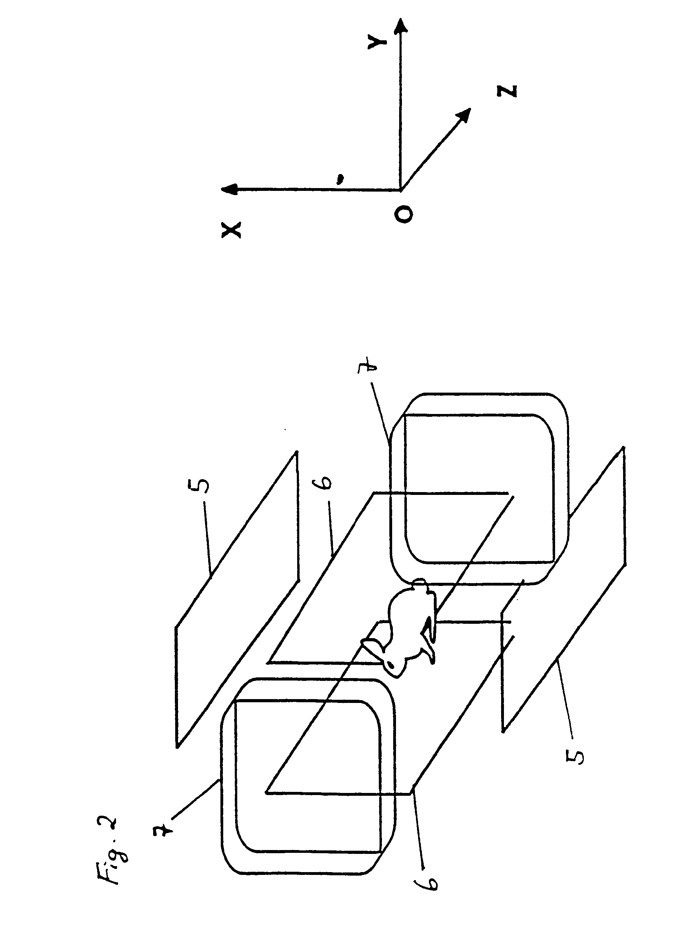

Aus Fig. 2 ist eine weitere bevorzugte Spulen bzw. Magnetfeldanordnung der erfindungsgemäßen Vorrichtung zu erkennen. Gegenüber Fig. 1 wird das Magnetfeld B₀ nicht durch einen Permanentmagneten 1 erzeugt, sondern durch eine Helmholtzspulenpaar 7. Durch den Einsatz von Helmholtzspulen im Rahmen dieser Ausführungsform kann auf die Hilfsspule 4 aus Fig. 1 verzichtet werden. Die durchzuführende Feldverschiebung im Rahmen eines möglichen schnellen adiabatischen Durchlaufs kann durch eine geeignete Variation des Spulenstroms erzielt werden.

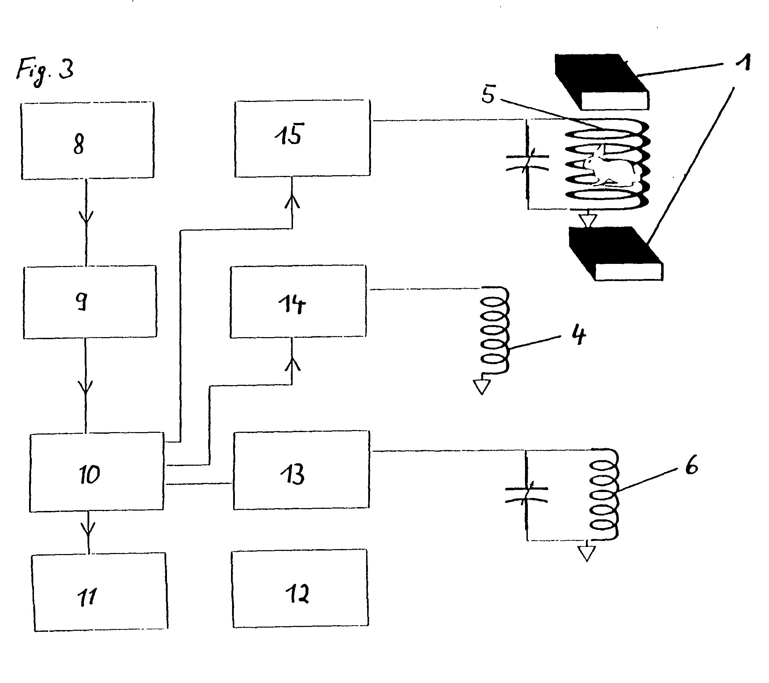

Aus Fig. 3 ist ein Blockschaltbild zu entnehmen, das schematisch die Funktionsgruppen einer Behandlungsvorrichtung zeigt, bei der beispielhaft eine erfindungsgemäße Vorrichtung zur Behandlung mit magnetischen Feldern zum Einsatz kommt. Im Rahmen dieses Ausführungsbeispiels umfaßt die Vorrichtung einen sog. Kartenleser 8, der zur Aufnahme und zum Erkennen von mit Magnetstreifen und/oder beschreibbare Speicherchips bestückten Chipkarten geeignet ist, wie sie beispielsweise heute üblicherweise von Krankenkassen bereits an die Patienten ausgegeben werden. Die vorliegende Vorrichtung ist dadurch in der Lage automatisiert Informationen über Patienten bzw. zur Patientenbehandlung aufzunehmen und im Rahmen der erfindungsgemäßen Vorrichtung einer Weiterverarbeitung zuzuführen. Solche Daten können unter anderem sein, eine Historie über den bisherigen Behandlungsverlauf, Angaben über die Behandlungsdauer, die Anzahl der verordneten oder bezahlten Behandlungen, individuelle Angaben zur Behandlungsintensität und was sehr wichtig ist die Wiederholungsrate mit der die Spinresonanzanwendung durchgeführt werden sollen. Dabei ist für den Fachmann, der auf dem Gebiet tätig ist, leicht ersichtlich, daß die beschriebene Datenübermittlung bzw. Zugangsberechtigung auch in anderer Form durchführbar ist. Demzufolge ist beispielsweise auch eine Übermittlung von zentraler Stelle aus, mittels geeigneter und dem Fachmann geläufiger Schnittstellen möglich.

Die gelesenen Daten werden dann an eine Mikrokontrollersteuerung 9, die auch sämtliche Steuereinrichtungen umfaßt, weitergegeben. Die Mikroprozessoreinrichtung steuert unter anderem den Spinresonanzprozess, die Wiederholungsrate bzw. die Abfolge von mehreren Spinabsorptionsprozessen und deren Wirksamkeit. Ferner kann mit der benannten Mikrokontrollereinrichtung 9 beim behandeln von mehreren Körperteilen oder eines Körperbereichs mittels der Einrichtung beispielsweise auch der Vorschub einer Behandlungsliege innerhalb der Aktiv-Zone 2 (Fig. 1) der erfindungsgemäßen Vorrichtung gesteuert werden. Beispielhaft ist in Fig. 3 die Verbindung der Mikrokontrollersteuerung zum erfindungsgemäßen Funktionsgenerator 10 dargestellt. Der Funktionsgenerator 10 erzeugt unter anderem in den entsprechenden Schwingkreisen geeignete Frequenzen zur definierten Erzeugung von Kernspinresonanzen, die durch die Mikrokontrollereinrichtung gesteuert und überwacht werden. Ferner umfaßt die erfindungsgemäße Vorrichtung eine Anzeige 11, durch die beispielsweise der Patient oder eine Aufsichtsperson den Vorgang der Kerspinresonanzbehandlung genau zu überwachen vermag.

## Patentansprüche

1. Vorrichtung zur Verwendung für die therapeutische Behandlung eines in einer Aktiv-Zone (2) befindlichen Teils eines biologischen Körpers (3) mit magnetischen Feldern, umfassend einen Magneten (1, 4, 7) zur Erzeugung eines Magnetfeldes und ein Sendespulensystem (5) zur Erzeugung eines magnetischen Wechselfeldes senkrecht zu dem Magnetfeld, wobei mittels der Vorrichtung Ker spinresonanzen innerhalb des zu behandelnden biologischen Körpers (3) erzeugbar sind,
**gekennzeichnet durch**
ein weiteres Spulensystem (6), welches als Detektionsspulensystem verwendbar ist und eine Steuerelektronik (9), welche eine Meßeinrichtung umfaßt, **durch** die, die **durch** die Kernspinresonanz an das zu behandelnde Gewebe bzw. den Körperteil abgegebene Energie, meßbar ist.

2. Vorrichtung nach Anspruch 1, **dadurch gekennzeichnet, daß**
- die Steuerelektronik (9) zum Ansteuern des Magneten (1, 4, 7) und des Sendespulensystems (5) zum Erzeugen einer Kernspinresonanz in zumindest einem Teil eines biologischen Körpers (3) ausgebildet ist und
- die Vorrichtung eine Einrichtung zum wiederholten Ansteuern der Steuerelektronik (9) zum Erzeugen einer Abfolge von Kernspinresonanzen umfaßt.

3. Vorrichtung nach Anspruch 2, **dadurch gekennzeichnet, daß** die durch die Einrichtung zum wiederholten Ansteuern der Steuerelektronik (9) erzeugte Abfolge von Kernspinresonanzen mittels der Spin-Gitter-Relaxationszeit T₁ des zumindest einen Teils des biologischen Körpers (3) bestimmbar ist und vorzugsweise eine Wiederholungsrate von ca. 3T₁ aufweist.

4. Vorrichtung nach einen der vorhergehenden Ansprüche, **dadurch gekennzeichnet, daß** das durch das Sendespulensystem (5) erzeugte magnetische Wechselfeld definierte Fourier-Komponenten umfaßt.

5. Vorrichtung nach einem der vorhergehenden Ansprüche, **dadurch gekennzeichnet, daß** die Achse des weiteren Spulensystems senkrecht zum Magnetfeld des Magneten (1, 4, 7) und senkrecht zur Achse des Sendespulensystems (5) zum Erzeugen eines Wechselfeldes ausgebildet ist.

6. Vorrichtung nach einem der vorhergehenden Ansprüche, **dadurch gekennzeichnet, daß** die Steuerelektronik (9) eine Auswerteeinrichtung umfaßt, mittels der die Spin-Gitter-Relaxations-Zeit T₁ bestimmbar ist.

7. Vorrichtung nach einem der vorhergehenden Ansprüche, **dadurch gekennzeichnet, daß** der Magnet (1, 4) eine Helmholtzspule (7) umfaßt.

8. Vorrichtung nach einem der vorhergehenden Ansprüche, **dadurch gekennzeichnet, daß** der Magnet (4, 7) einen Hartferrit Magneten (1) umfaßt.

9. Vorrichtung nach einem der vorhergehenden Ansprüche, **dadurch gekennzeichnet, daß** das Magnetfeld des Magneten (1, 4, 7) eine Abweichung von bis zu ca. ± 0,5 Gauß aufweisen kann.

10. Vorrichtung nach einem der vorhergehenden Ansprüche, **dadurch gekennzeichnet, daß** der Magnet (1, 7) eine zusätzliche Hilfsspule (4) zum Erzeugen eines magnetischen Hilfsfeldes aufweist und das Hilfsfeld das Magnetfeld des Magneten (1, 7) überlagert.

11. Vorrichtung nach Anspruch 10, **dadurch gekennzeichnet, daß** die Steuerelektronik (9) eine Steuereinrichtung zum Ansteuern der Hilfsspule (4) umfaßt.

## Claims

1. Device for use in the therapeutic treatment of a part of a biological body (3) located in an active zone (2) with magnetic fields, comprising a magnet (1, 4, 7) for generating a magnetic field and a transmitting coil system (5) for generating an alternating magnetic field transversely to the magnetic field wherein nuclear magnetic resonance can be generated within the biological body (3) to be treated by means of the device,
**characterised by**
a further coil system (6) which can be used as a detection coil system and an electronic control unit (9) which comprises a measuring device, through which the energy emitted through the nuclear magnetic resonance to the tissue or the body part to be treated can be measured.

2. Device according to claim 1, **characterised in that**
- the electronic control unit (9) is designed to control the magnet (1, 4, 7) and the transmitting coil system (5) for generating nuclear magnetic resonance in at least one part of a biological body (3) and
- the device comprises a device for repetitive control of the electronic control unit (9) for generating a nuclear magnetic resonance sequence.

3. Device according to claim 2, **characterised in that** the nuclear magnetic resonance sequence generated by the device for repetitive control of the electronic control unit (9) can be determined by means of the spin-lattice relaxation time T₁ of the at least one part of the biological body (3) and preferably has a repetition rate of approximately 3T₁.

4. Device according to one of the preceding claims, **characterised in that** the alternating magnetic field generated by the transmitting coil system (5) comprises defined Fourier components.

5. Device according to one of the preceding claims, **characterised in that** the axis of the further coil system is formed perpendicularly to the magnetic field of the magnet (1, 4, 7) and perpendicularly to the axis of the transmitting coil system (5) for generating an alternating field.

6. Device according to one of the preceding claims, **characterised in that** the electronic control unit (9) comprises an evaluating device, by means of which the spin-lattice relaxation time T₁ can be determined.

7. Device according to one of the preceding claims, **characterised in that** the magnet (1, 4) comprises a Helmholtz coil (7).

8. Device according to one of the preceding claims, **characterised in that** the magnet comprises a hard ferrite magnet (1).

9. Device according to one of the preceding claims, **characterised in that** the magnetic field of the magnet (1, 4, 7) can have a deviation of up to approximately +/-0.5 gauss.

10. Device according to one of the preceding claims, **characterised in that** the magnet (1, 7) comprises an additional auxiliary coil (4) for generating an auxiliary magnetic field and the auxiliary field overlies the magnetic field of the magnet (1, 7).

11. Device according to claim 10, **characterised in that** the electronic control unit (9) comprises a control device for controlling the auxiliary coil (4).

## Revendications

1. Dispositif destiné à être utilisé pour le traitement thérapeutique avec des champs magnétiques d'une partie d'un corps biologique (3) se trouvant dans une zone active (2), comportant un aimant (1, 4, 7) pour la génération d'un champ magnétique, et un système de bobines émettrices (5) pour la génération d'un champ magnétique alternatif perpendiculairement au champ magnétique, des résonances magnétiques nucléaires pouvant être générées à l'intérieur du corps biologique (3) au moyen du dispositif,
**caractérisé par**
un autre système de bobines (6) pouvant être utilisé en tant que système de bobines de détection, et par une électronique de commande (9) comportant un dispositif de mesure, avec lequel peut être mesurée l'énergie délivrée au tissu ou à la partie corporelle par la résonance magnétique nucléaire.

2. Dispositif selon la revendication 1, **caractérisé en ce que**
- l'électronique de commande (9) pour la commande de l'aimant (1, 4, 7) et du système de bobines émettrices (5), est réalisée pour la génération d'une résonance magnétique nucléaire dans au moins une partie d'un corps biologique (3), et
- **en ce que** le dispositif comporte un système pour la commande réitérée de l'électronique de commande (9) destinée à la génération d'une série de résonances magnétiques nucléaires.

3. Dispositif selon la revendication 2, **caractérisé en ce que** la série de résonances magnétiques nucléaires générée par le système pour la commande réitérée de l'électronique de commande (9) peut être déterminée au moyen du temps de relaxation longitudinale (spin-réseau) T₁ de l'au moins: une partie du corps biologique (3), et présente de préférence un taux de répétitivité d'environ de 3T₁.

4. Dispositif selon l'une des revendications précédentes, **caractérisé en ce que** le champ magnétique alternatif généré par le système de bobines émettrices (5) comprend des composantes définies de Fourier.

5. Dispositif selon l'une des revendications précédentes, **caractérisé en ce que** l'axe de l'autre système de bobines est agencé perpendiculairement au champ magnétique de l'aimant (1, 4, 7) et perpendiculairement à l'axe du système de bobines émettrices (5) pour la génération d'un champ alternatif.

6. Dispositif selon l'une des revendications précédentes, **caractérisé en ce que** l'électronique de commande (9) comporte un dispositif d'évaluation, au moyen duquel le temps de relaxation T₁ de la grille de spin peut être déterminé.

7. Dispositif selon l'une des revendications précédentes, **caractérisé en ce que** l'aimant (1, 4) comporte une bobine de Helmholtz (7).

8. Dispositif selon l'une des revendications précédentes, **caractérisé en ce que** l'aimant (4, 7) comporte un aimant (1) à ferrite dure .

9. Dispositif selon l'une des revendications précédentes, **caractérisé en ce que** le champ magnétique de l'aimant (1, 4, 7) peut présenter un écart pouvant aller jusqu'à environ ± 0,5 Gauss.

10. Dispositif selon l'une des revendications précédentes, **caractérisé en ce que** l'aimant (1, 7) comporte une bobine auxiliaire supplémentaire (4) pour la génération d'un champ magnétique auxiliaire, et **en ce que** le champ auxiliaire est superposé au champ magnétique de l'aimant (1, 7).

11. Dispositif selon la revendication 10, **caractérisé en ce que** l'électronique de commande (9) comporte un dispositif de commande pour la commande de la bobine auxiliaire (4).
